(19) Europäisches Patentamt
European Patent Office
Office européen des brevets

(11) Numéro de publication: **0 070 753**
**A1**

# (12) DEMANDE DE BREVET EUROPEEN

(21) Numéro de dépôt: **82401201.7**

(22) Date de dépôt: **29.06.82**

(51) Int. Cl.³: **C 07 D 453/02**, A 61 K 31/54

(30) Priorité: **10.07.81 FR 8113602**

(43) Date de publication de la demande: **26.01.83**
**Bulletin 83/4**

(84) Etats contractants désignés: **BE CH DE FR GB IT LI LU NL SE**

(71) Demandeur: **PHARMINDUSTRIE, 35 Quai du Moulin de Cage, F-92231 Gennevilliers (FR)**

(72) Inventeur: **Dubroeucq, Marie-Christine, 13 Villa Malleville, F-95880 Enghien-les-Bains (FR)**
Inventeur: **Rataud, Jean Ernest Marcel André, 115, rue de Reuilly, F-75012 Paris (FR)**

(74) Mandataire: **Houssin, Jean, P C U K Produits Chimiques Ugine Kuhlmann Service Propriété Industrielle Tour Manhattan - Cedex 21, F-92087 Paris La Defense 2 (FR)**

## (54) (Aza-1 bicyclo-(2,2,2) octyl-3) -10 10H phénothiazine-sulfonamide-2 et dérivés.

(57) Les [aza-1-bicyclo-(2,2,2) octyl-3]-10 10H phénothiazine-sulfonamides-2 de formule: (I)

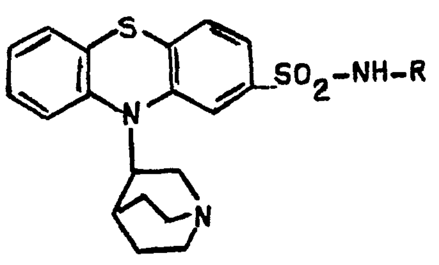

dans laquelle R représente l'atome d'hydrogène ou un groupe alkyle comprenant de 1 à 3 atomes de carbone.

Ces composés sont utilisables comme médicaments pour le traitement des ulcères duodénaux et gastriques.

EP 0 070 753 A1

[aza-1 bicyclo-(2,2,2) octyl-3] -10 10H phénothiazine-sulfonamide-2 et dérivés

La présente invention a pour objet l'[aza-1 bicyclo-(2,2,2) octyl-3] -10 10H phénothiazine-sulfonamide-2 et ses dérivés N-mono-alkylès, utilisables comme médicaments notamment pour le traitement des ulcères gastriques et duodénaux.

Ces composés peuvent être représentés par la formule générale :

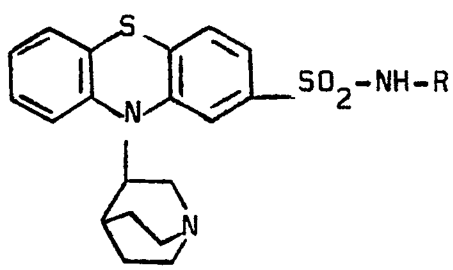

(I)

dans laquelle R représente l'atome d'hydrogène ou un groupe alkyle comprenant de 1 à 3 atomes de carbone.

Les composés selon l'invention peuvent être préparés à partir de la N,N-diméthyl [aza-1 bicyclo-(2,2,2) octyl-3] -10 10 H phénothiazinesulfonamide-2, dont la préparation est décrite dans le certificat d'addition français 75 23340 (2.318.638). Le procédé selon l'invention comprend quatre étapes, qui peuvent être représentées comme suit :

a)

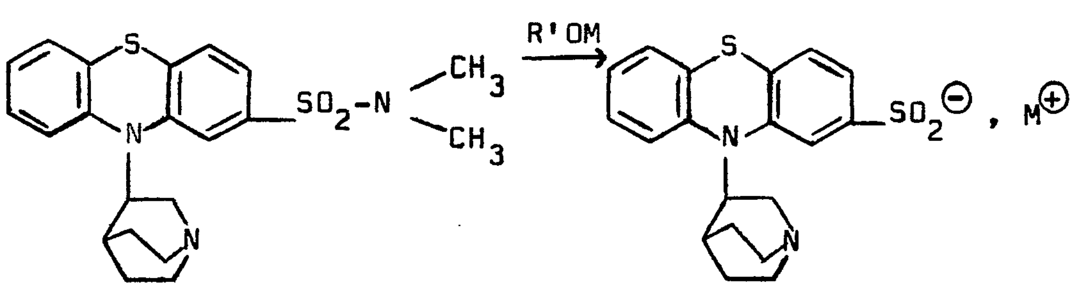


(II)

dans la formule (II) M représente un atome de métal alcalin tel que sodium ou potassium. R'OM représente un alcoolate de métal alcalin, comprenant 1 à 7 atomes de carbone.

b) (II) 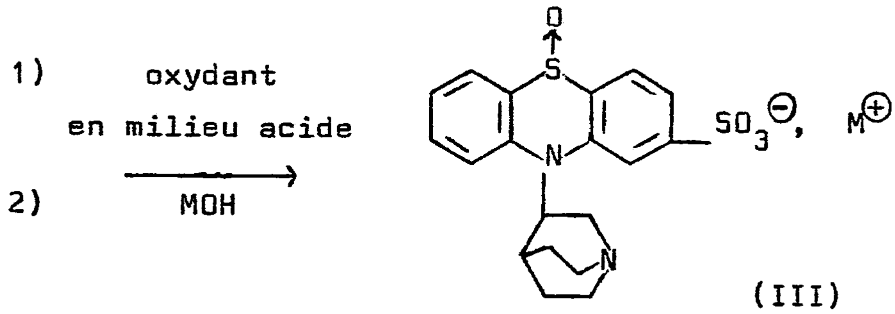


(III)

c) (III) 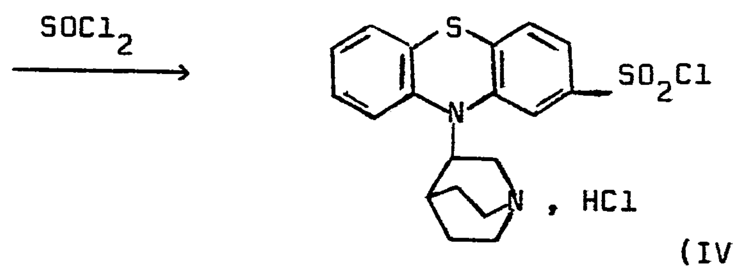


(IV)

d) (IV) $R-NH_2$ (I)

Pour effectuer la réaction a) on traite la N,N diméthyl [aza-1 bicyclo-(2,2,2) octyl-3]-10 10H phénothiazine-sulfonamide-2 par un alcoolate au sein d'un solvant : tel que le tétrahydrofuranne au reflux ou un alcool à haut point d'ébullition tel que l'alcool isoamylique au reflux ou encore un solvant aprotique dipolaire tel que le diméthylsulfoxyde à une température supérieure à 80°C. Un procédé avantageux consiste à utiliser le tertiobutylate de potassium au sein du diméthylsulfoxyde à 100°C.

L'étape b) consiste à oxyder le groupement sulfinate en groupement sulfonate. Au cours de cette réaction s'effectue également l'oxydation en sulfoxyde de l'atome de soufre du cycle phénothiazine. Pour effectuer ces réactions on traite le composé de formule (II) par un oxydant en

milieu aqueux en présence d'au moins 2 équivalents d'un acide fort (par exemple l'acide méthanesulfonique). On utilise à cet effet les oxydants connus permettant de transformer un acide sulfinique en acide sulfonique (cf. Comprehensive Organic Chemistry, Vol. 3 p. 317 K.K. ANDERSEN. Sulphinic acids and their Derivatives - Pergamon Presse 1979). Une méthode avantageuse consiste à utiliser le métapériodate de sodium dans l'eau, en présence d'acide méthanesulfonique, à température ambiante. En fin de réaction, on alcalinise au moyen d'un hydroxyde de métal alcalin MOH ; on obtient finalement un sel alcalin de l'acide [aza-1 bicyclo-(2,2,2) octyl-3]-10 10H phénothiazine oxyde-5 sulfonique-2 de formule (III).

Dans l'étape c) le composé de formule (III) est soumis à l'action du chlorure de thionyle qui d'une part réduit la fonction sulfoxyde, régénérant ainsi l'atome de soufre du cycle phénothiazine et d'autre part transforme le groupement sulfonate en groupement sulfochlorure. On opère au sein d'un solvant inerte à une température comprise entre 60 et 150°C et de préférence en présence de diméthylformamide comme catalyseur.

Une méthode avantageuse consiste à utiliser le toluène au reflux contenant de 0,5 à 2 % de diméthylformamide.

L'acide chlorhydrique dégagé au cours de la réaction salifie le composé formé que l'on obtient finalement sous forme de chlorhydrate en mélange avec le chlorure de métal alcalin formé. Ce mélange est utilisé tel quel dans l'étape suivante.

Dans l'étape d) le produit obtenu est traité par l'amine appropriée de formule $R-NH_2$ en excès. Dans le cas de l'ammoniac (R=H) on utilise avantageusement l'ammoniac liquide à sa température d'ébullition. Dans le cas d'alkylamines (R = alkyle) on opère avantageusement au sein d'un solvant inerte tel qu'un hydrocarbure aromatique, par exemple, le benzène ou le toluène à température ambiante.

Les mélanges réactionnels obtenus par le procédé décrit précédemment sont traités selon des méthodes classiques, (évaporation, extraction à l'aide d'un solvant, distillation, cristallisation, chromatographie, etc.) afin d'isoler les nouveaux composés de formule (I) à l'état pur. Dans le procédé selon l'invention les composés de formule (II), (III) et (IV) sont également des produits nouveaux.

Les composés de formule (I) sous forme de base libre peuvent éventuellement être transformés en sels d'addition avec un acide minéral ou organique par action d'un tel acide au sein d'un solvant approprié.

Les exemples suivants illustrent l'invention sans la limiter.

<u>Exemple</u> 1 : [AZA-1 BICYCLO (2,2,2) OCTYL-3]-10 10H PHENOTHIAZINESULFONAMIDE-2.

a) [AZA-1 BICYCLO (2,2,2) OCTYL-3]-10 10H PHENOTHIAZINE-SULFINATE-2 DE POTASSIUM.

A une solution chauffée à 60°C de 4,15 g de N,N diméthyl [aza-1 bicyclo-(2,2,2) octyl-3] -10 10H-phénothiazine-sulfonamide-2 dans 60 ml de diméthylsulfoxyde on ajoute

4,48 g de tertiobutylate de potassium. Le mélange est agité et chauffé à 100°C pendant 1 heure. Après refroidissement à la température ambiante on ajoute quelques gouttes d'eau pour détruire l'excès de tertiobutylate de potassium. La solution est fixée sur une colonne de silice (500 g) ; on élue avec du méthanol. Les fractions de tête contiennent le diméthylsulfoxyde ; on recueille ensuite le produit attendu. On évapore le méthanol et le résidu est repris par 50 ml d'éthanol. Les cristaux blancs formés sont essorés et séchés. On obtient 2 g d'[aza-1 bicyclo-(2,2,2) octyl-3] -10 10H phénothiazinesulfinate de potassium fondant au-dessus de 250°C. Spectre I.R. (pastille KBr) : 980 et 1.020 $cm^{-1}$ ($-SO_2^-$).

b) [aza-1 bicyclo-(2,2,2) octyl-3] -10 10H phénothiazine oxyde-5 sulfonate-2 de potassium.

On agite pendant 16 heures à température ambiante un mélange de 6 g d' [aza-1 bicyclo-(2,2,2) octyl-3] -10 10H phénothiazinesulfinate-2 de potassium, 33,3 ml d'une solution aqueuse 1,25 N d'acide méthanesulfonique 8,67 g de métapériodate de sodium et 300 ml d'eau. En fin de réaction on obtient une solution rouge avec un léger insoluble qui est éliminé par filtration. Le filtrat est alcalinisé à pH = 11 par addition d'hydroxyde de potassium puis évaporé à sec sous pression réduite. Le résidu est fixé sur une colonne de silice et élué par du méthanol. Après évaporation des fractions éluées on obtient 7,2 g d' [aza-1 bicyclo-(2,2,2) octyl-3] -10 10H phénothiazine oxyde-5 sulfonate-2 de potassium, fondant au-dessus de 250°C. Spectre I.R. (pastille KBr) ; 1.020 et 1.200 $cm^{-1}$ ($-SO_3^-$).

c) [aza-1 bicyclo-(2,2,2) octyl-3] -10 10H phénothiazine sulfonamide-2.

A une suspension agitée de 6,6 g d' [aza-1 bicyclo-(2,2,2) octyl-3] -10 10H phénothiazine oxyde-5 sulfonate-2 de potassium dans 150 ml de toluène on ajoute goutte-à-goutte 7 ml de chlorure de thionyle. On chauffe au reflux pendant 30 minutes, refroidit à 50°C et introduit goutte-à-goutte une solution de 2 ml de diméthylformamide dans 30 ml de toluène. La suspension jaune obtenue est chauffée au reflux pendant 30 minutes puis le solvant et le chlorure de thionyle en excès sont évaporés sous pression réduite. Le résidu est mis en suspension dans 100 ml de toluène et le toluène est ensuite évaporé. On obtient 7 g d'un solide jaune constitué d'un mélange de chlorure d' [aza-1 bicyclo-(2,2,2) octyl-3] -10 10H phénothiazine-sulfonyle -2 sous forme de chlorhydrate et de chlorure de potassium.

d) 1 g de ce mélange est introduit dans 100 ml d'ammoniac liquide à -30°C. On laisse l'ammoniac s'évaporer lentement. Le résidu est repris par 50 ml de chloroforme et 50 ml d'eau. La phase organique est décantée, séchée sur sulfate de magnésium et évaporée. Le résidu est fixé sur une colonne de silice et élué par du méthanol. On obtient ainsi 0,35g qui sont mis en suspension dans 5 ml d'oxyde d'isopropyle. Les cristaux sont essorés et séchés ; on obtient 0,31 g d' [aza-1 bicyclo-(2,2,2) octyl-3]-10 10H phénothiazinesulfonamide-2, fondant au-dessus de 260°C.

<u>Exemple 2</u> : N-METHYL [AZA-1 BICYCLO-(2,2,2) OCTYL-3]-10 10H PHENOTHIAZINESULFONAMIDE-2

A une suspension agitée de 6,6 g d' [aza-1 bicyclo-(2,2,2) octyl-3] -10 10H phénothiazine oxyde-5 sulfonate-2 de potassium (préparé à l'exemple 1) dans 150 ml de toluène on ajoute goutte-à-goutte 7 ml de chlorure de thionyle. On chauffe

au reflux pendant 30 minutes, refroidit à 50°C et introduit goutte-à-goutte une solution de 2 ml de diméthylformamide dans 30 ml de toluène. La suspension jaune obtenue est chauffée au reflux pendant 30 minutes puis le solvant et le chlorure de thionyle en excès sont évaporés sous pression réduite. Le résidu est mis en suspension dans 100 ml de toluène et le toluène est ensuite évaporé. On obtient ainsi 7 g d'un solide jaune constitué d'un mélange de chlorure d' [aza-1 bicyclo-(2,2,2) octyl-3] -10 10H phénothiazinesulfonyle-2 sous forme de chlorhydrate et de chlorure de potassium.

5,5 g de ce mélange sont introduits dans 100 ml de toluène. On ajoute goutte-à-goutte à la suspension obtenue et en agitant 100 ml d'une solution à 10 % en poids de méthylamine dans le toluène. Le mélange réactionnel est agité 16 heures à température ambiante puis le solvant est évaporé sous pression réduite. Le résidu est fixé sur une colonne de silice et élué par un mélange contenant 9 parties en volume de chloroforme et 1 partie en volume de diéthylamine. On obtient ainsi 3,5 g de produit qu'on recristallise dans 50 ml d'acétonitrile. On obtient finalement 2,3 g de N-méthyl [aza-1 bicyclo-(2,2,2) octyl-3] -10 10H phénothiazinesulfonamide-2 fondant à 255°C.

Propriétés pharmacologiques

L'activité antisécrétoire des composés selon l'invention de formule (I) a été étudiée sur l'hypersécrétion gastrique stimulée par la pentagastrine chez le chien porteur d'une poche de Heidenhein.

Trois chiens "mongrels" porteurs d'une poche de Heidenhein, à jeun depuis 18 heures, reçoivent une perfusion veineuse

de 4 mg/kg/heure de pentagastrine sous un volume de 30 ml/heure pendant 4 heures. La sécrétion de la poche est recueillie dans un flacon qui est changé toutes les 15 minutes. Une heure et demie après le début de la perfusion, le produit à étudier est administré par voie orale dans une gélule h°000. La dose efficace 50 % (DE 50) est la dose qui diminue de 50 % le débit acide horaire de la 3ème ou 4ème heure de l'expérimentation par rapport au débit acide de la deuxième heure de la perfusion. Les doses sont exprimées en produit sous forme de base.

A titre d'exemple, on trouvera dans le tableau ci-dessous les résultats obtenus avec le produit de l'exemple 2.

| PRODUITS | $DE_{50}$ en mg/kg p.o. |
|---|---|
| CIMETIDINE | 2,3 |
| N,N DIMETHYL [AZA-1 BICYCLO(2,2,2) OCTYL-3] -10 10H PHENOTHIAZINE-SULFONAMIDE-2 | 1,25 |
| EXEMPLE 2 | 0,7 |

Les produits de l'invention sont de puissants inhibiteurs de sécrétion gastrique. En particulier le produit de l' exemple 2 est plus actif que la cimétidine.

Propriétés toxicologiques

Les toxicités aiguës des composés selon l'invention ont été déterminés chez la souris mâle $CD_1$ (Charles RIVER) par voie orale. Les $DL_{50}$ ont été calculées après 3 jours d'observation, par la méthode cumulative de J.J. REED et H. MUENCH (Amer. J. Hyg. 1938, 27, 493).

Les composés se comportent comme des substances relativement peu toxiques chez la souris, puisque les $DL_{50}$ des composés se situent entre 300 et 1000 mg/kg.

Utilisation thérapeutique

Les composés selon l'invention et leurs sels pharmaceutiquement acceptables peuvent être utilisés en thérapeutique humaine, sous forme de comprimés, capsules, gélules, suppositoires, solutions ingérables ou injectables, etc. comme inhibiteurs de sécrétion gastrique pour le traitement des ulcères duodénaux et gastriques.

La posologie dépend des effets recherchés et de la voie d'administration utilisée. Par exemple, par voie orale, elle peut être comprise entre 50 et 500 mg de substance active par jour, avec des doses unitaires allant de 10 à 100 mg.

Revendications

1) Les [aza-1 bicyclo-(2,2,2) octyl-3] -10 10H phénothiazine sulfonamides-2 de formule :

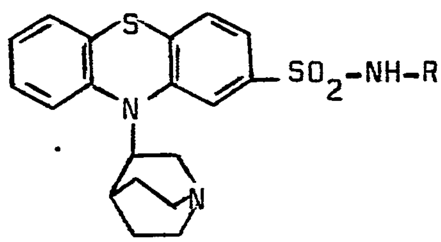

(I)

dans laquelle R représente l'atome d'hydrogène ou un groupe alkyle comprenant 1 à 3 atomes de carbone.

2) Procédé pour la préparation des composés de formule (I) caractérisé en ce que l'on fait réagir la N,N diméthyl [aza-1 bicyclo-(2,2,2) octyl-3] -10 10H phénothiazine-sulfonamide-2 avec un alcoolate de métal alcalin au sein d'un solvant, traite le sulfinate ainsi obtenu par un oxydant en milieu acide, alcalinise au moyen d'un hydroxyde de métal alcalin, soumet le sel de l'acide [aza-1 bicyclo-(2,2,2) octyl-3] -10 10H phénothiazine oxyde-5 sulfonique-2 ainsi obtenu à l'action du chlorure de thionyle et fait réagir le mélange réactionnel obtenu comprenant le chlorure de sulfonyle avec un composé de formule $R-NH_2$, R ayant les mêmes significations que sous 1.

3) Les acides [aza-1 bicyclo-(2,2,2) octyl-3] -10 10H phénothiazine-sulfinique-2 et [aza-1 bicyclo-(2,2,2) octyl-3] -10 10H phénothiazine-oxyde-5 sulfonique-2, leurs sels alcalins et le chlorure d'[aza-1 bicyclo-(2,2,2) octyl-3] -10 10H phénothiazinesulfonyle-2, formés dans le procédé défini sous 2.

4) En tant qu'agents inhibiteurs de la sécrétion gastrique, utilisables comme médicaments, spécialement pour le traitement des ulcères duodénaux et gastriques, les composés revendiqués selon la revendication 1 et leurs sels avec les acides pharmaceutiquement acceptables.

0070753

Office européen des brevets

# RAPPORT DE RECHERCHE EUROPEENNE

Numéro de la demande

EP 82 40 1201

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl. 3) |
|---|---|---|---|
| D,Y | FR-A-2 318 638 (SOGERAS)<br>* en entier *<br>--- | 1,4 | C 07 D 453/02<br>A 61 K 31/54 |
| Y | FR-A-2 034 605 (SOGERAS)<br>* revendications *<br>----- | 1,4 | |
| | | | **DOMAINES TECHNIQUES RECHERCHES (Int. Cl. 3)** |
| | | | C 07 D 453/00<br>A 61 K 31/00 |

Le présent rapport de recherche a été établi pour toutes les revendications

| Lieu de la recherche | Date d'achèvement de la recherche | Examinateur |
|---|---|---|
| LA HAYE | 01-09-1982 | NUYTS A.M.K.A. |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

& : membre de la même famille, document correspondant

OEB Form 1503 03.82